(19) Europäisches Patentamt
European Patent Office
Office européen des brevets

(11) **EP 4 122 479 A1**

(12) **EUROPEAN PATENT APPLICATION**
published in accordance with Art. 153(4) EPC

(43) Date of publication:
**25.01.2023 Bulletin 2023/04**

(21) Application number: **21772338.6**

(22) Date of filing: **12.03.2021**

(51) International Patent Classification (IPC):
**A61K 36/27** (2006.01)    **A61K 36/53** (2006.01)
**A61K 36/232** (2006.01)    **A61P 25/22** (2006.01)
**A23L 33/105** (2016.01)

(52) Cooperative Patent Classification (CPC):
**A23L 33/105; A61K 36/232; A61K 36/27;**
**A61K 36/53; A61P 25/22**

(86) International application number:
**PCT/KR2021/003081**

(87) International publication number:
**WO 2021/187809 (23.09.2021 Gazette 2021/38)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **16.03.2020 KR 20200031859**

(71) Applicant: **Natural Endotech Co., Ltd.**
**Gyeonggi-do 13486 (KR)**

(72) Inventors:
• **KIM, Jae Soo**
**Seongnam-si, Gyeonggi-do 13486 (KR)**

• **LEE, Yong Wook**
**Seongnam-si, Gyeonggi-do 13486 (KR)**
• **PARK, Chan sung**
**Seoul-Si (KR)**
• **OH, Joohyun**
**Hanam-Si (KR)**
• **KIM, Ji Min**
**Hanam-Si (KR)**
• **JO, Ha Neul**
**Seongnam-si (KR)**

(74) Representative: **Plougmann Vingtoft a/s**
**Strandvejen 70**
**2900 Hellerup (DK)**

(54) **TRANQUILIZING COMPOSITION**

(57)    The present invention relates to a composition for improving, preventing or treating an anxiety disorder, and a tranquilizing composition.

【FIG. 6】

EP 4 122 479 A1

Processed by Luminess, 75001 PARIS (FR)

## Description

**Technical field**

The present invention relates to a composition for improving, preventing or treating an anxiety disorder, and a tranquilizing composition.

**Background art**

Anxiety, which is a kind of defensive system for protecting the human body, activates the autonomic nervous system. Frequent anxiety is accompanied by tiredness, stress, overfatique, etc., and the sympathetic nervous system becomes overactive, which causes disturbance in lives and evolves into anxiety disorders. Anxiety disorders include post-traumatic stress disorder, obsessive-compulsive disorder, social phobia and generalized anxiety disorder, and anxiety involves other mental disorders such as depression or drug abuse. Particularly, social anxiety disorder and generalized anxiety disorder are not well known, but many people struggle thereagainst.

Anxiety disorders are a cluster of mental disorders characterized by various types of abnormal and morbid feelings of anxiety and fear which lead to impairment in everyday life. Fear and anxiety are normal emotional responses, but when excessive beyond the appropriate range, they cause mental pain and physical symptoms. The sympathetic nerve activated by anxiety causes the physical symptoms such as headache, fast heart rate, rapid breathing rate or gastrointestinal disorders. Those who have difficulty in carrying out their daily activities such as family life, work life or learning may be diagnosed with anxiety disorders. There are multiple forms of anxiety disorders with specific clinical definitions and diagnosis criteria. Disorders corresponding to anxiety disorders include panic disorder, specific phobia (acrophobia, homophobia, ophidiophobia, etc.), social phobia, obsessive-compulsive disorder, post-traumatic stress disorder, generalized anxiety disorder, and acute stress disorder. Anxiety disorders cover various and different metal disorders and are caused by a complex combination. Thus, it is difficult to define anxiety disorders in one word.

Generally, the causes of morbid anxiety may be lack or excess of neurotransmitters in the cerebral nerve which is in charge of emotions such as anxiety or depression, genetically inherited causes, functional or structural changes in the brain found from neuroimaging studies, social psychological aspects, and cognitive behavioral factors interpreting and determining past experience and current information. Particularly, accidents or catastrophes causing considerable psychological impacts are the main causes of post-traumatic stress disorder or acute stress disorder.

As anxiety is a normal psychological response, it is impossible to completely eliminate anxiety. Anxiety may be alleviated by treatment and patients may return to the normal state. In many cases, however, anxiety easily recurs and becomes chronic. Anxiety disorders are commonly accompanied by depression, and when patients frequently drink alcohol to reduce anxiety, addiction such as alcohol dependence may occur.

At present, representative antianxiety drugs commercially used are benzodiazepines, such as diazepam, oxazepam, prazepam, lorazepam, alprazolam, helazepam, or clonazepam. These drugs are mainly used for tranquilization and sleep induction.

The advantage of benzodiazepines is fast pharmacological effect, and benzodiazepines are most commonly prescribed in Korea. The mechanism of action of benzodiazepines has been known to enhance an affinity for gamma-aminobutyric acid (GABA) receptor, which is the representative inhibitory neurotransmitter in the central nervous system, and increase the frequency of the opening of the associated $Cl^-$ ion channel to increase the conductance of $Cl^-$ ions. Although benzodiazepines exhibit the effect immediately, they have a disadvantage of being habit-forming and addictive drugs. Thus, unless benzodiazepines are taken according to doctors' treatment, symptoms recur or withdrawal syndromes occur. It has been reported that other side effects include drowsiness, ataxia, orthostatic hypotension, respiratory depression, headache, chronic sleep disorder, liver disease, etc.

Meanwhile, buspirone recently draws attention as it is not a habit-forming and dependent drug although it has a somewhat slow treatment effect. Buspirone has been increasingly prescribed as it is not associated with impairment of cognitive ability such as memory and impairment of motor performance.

In addition, in some cases, a selective serotonin reuptake inhibitor (SSRI) is used as an antianxiety drug. However, it takes several weeks for the drug to show the effect. Also, the drug may cause side effects such as weight gain or sexual dysfunction, and only a third of the patients obtain the effect. For some patients, their anxiety disorders get worse due to SSRI. Therefore, there is a need for a drug with a novel structure having less side effects and causing no habit formation and addiction.

**Detailed description of invention**

**Technical task**

It is an object of the present invention to provide a pharmaceutical composition for preventing or treating an anxiety disorder.
It is another object of the present invention to provide a food composition for preventing or improving an anxiety disorder.
It is yet another object of the present invention to provide a pharmaceutical composition for tranquilizing.
It is yet another object of the present invention to provide a food composition for tranquilizing.

**Means for solving technical task**

The present invention relates to a composition for improving, preventing or treating an anxiety disorder, and a tranquilizing composition.
Hereinafter, the present invention will be described in more detail.
An embodiment of the present invention relates to a pharmaceutical composition for preventing or treating an anxiety disorder, comprising at least one extract selected from the group consisting of *Cynanchum wilfordii, Phlomis umbrosa* and *Angelica gigas.*
In the present invention, the anxiety disorder may be, but is not limited to, separation or isolation anxiety disorder, paranoia, panphobia, generalized obsessive-compulsive disorder, anxiety neurosis or panic disorder.
Another embodiment of the present invention relates to a pharmaceutical composition for tranquilizing, comprising at least one extract selected from the group consisting of *Cynanchum wilfordii, Phlomis umbrosa* and *Angelica gigas.*
In the present invention, the pharmaceutical composition for tranquilizing may be for treating or preventing bipolar disorder, neurasthenia or schizophrenia, but is not limited thereto.
*Cynanchum wilfordii,* whose original plant is *Cynanchum wilfordii (Maximowicz) Hemsley,* is a vine plant growing in the sunny grass at the bottom of the mountain or on the slope by the sea. The root of the plant is harvested during the fall and winter, and dried for being used as a medicine. *Cynanchum wilfordii* has a sweet and slightly bitter taste and has a somewhat warm energy. This medicine reinforces the liver and kidney for strengthening the bones and muscles, improves the digestive organs, and has detoxifying properties in oriental medicine. Thanks to these effects, *Cynanchum wilfordii* may be used for the liver and kidney yin deficiency syndrome, erectile dysfunction, nocturnal emission, sore waist and knees, spleen deficiency, abdominal fullness, diarrhea, gray hairs growing early for one's age, or deficient lactation after delivery.
*Phlomis umbrosa* is a perennial herbaceous plant in the *Labiatae* family, with the binomial name of *Phlomis umbrosa* TURCZ. As one of medicinal herbs, it has been originally obtained by drying the root of teasel, but at present, teasel is rare. Thus, *Phlomis umbrosa* in the *Labiatae* family is replaced therewith. The name *Phlomis umbrosa* is given because the plant treats bone fractures well. The ingredients thereof are alkaloids, essential oil, vitamin E, etc. The medical properties thereof are mild and have a bitter taste. The plant has the effect on lumbar pain caused by weak liver or kidney, deficient skeletal structure of the legs, and unsmooth muscular motion. The plant also has effect on arthritis and rheumatism, and is used for the intervertebral discs and bruises. For women, the plant may prevent miscarriage when taken during pregnancy, and has the effect of stopping bleeding when there is a large amount of menstrual blood or there is severe uterine bleeding. For the elderly, the plant is commonly used for walking disability caused by the weak lower body, or numbness and pain caused by a failure to bend and stretch the body. It would be preferable to avoid the use of the plant along with *Rehmannia glutinosa,* and the plant is not used for dysentery. The representative prescription of *Phlomis umbrosa* is provided in the form of pills. The young shoot is eaten as seasoned vegetables.
*Angelica* refers to the root of dried *Angelica gigas Nakai* in the family Umbeliferous in Korea and refers to the root of *Angelica sinensis (Oliv.) Diels* in the family Umbeliferous in China. In Japan, the root of *Angelica acutiloba (Siebold. & Zucc.) Kitag.* or *Angelica acutiloba (Siebold. & Zucc.) Kitag. var. sugiyamae Hikino* in the family Umbeliferous is dried and used. The Korean name of *Angelica,* 'Danggui,' has the meaning of hoping to return home for sure. This comes from the Chinese custom that wives put *Angelica* in the arms of their husbands going to the war, believing that *Angelica* would restore their husbands' energy and send their husbands back home when they ran out of stream in the battle. According to one version, the name has the meaning "this medicine returns the energy and blood back." The medicine has warm energy and a sweet and spicy taste. Generally, *Angelica gigas* has less sweet taste and stronger spicy taste than *Angelica sinensis* or *Angelica acutiloba. Angelica* has the main effect of nourishing the blood when the blood is not sufficient. *Angelica* made from the root of *Angelica sinensis* or *Angelica acutiloba* has excellent blood nourishing effect, whereas *Angelica* made from the root of *Angelica gigas* has excellent blood circulation effect rather than blood nourishing effect, and has strong anticancer effect and blood pressure drop action. Pharmacologically, *Angelica* promotes the blood flow of the coronary arteries and vigorously produces erythrocytes. *Angelica gigas* is also called Todanggui, Sunggeomcho, Joseon Danggui. *Angelica sinensis* is also called Danggui, Mungui, Geongui, Daegeun, Sangma, and Jiseonwon. *Angelica acutiloba* is also called Ildanggui.

In the present invention, the composition may comprise at least one extract selected from the group consisting of *Cynanchum wilfordii, Phlomis umbrosa* and *Angelica gigas.* For example, the composition may comprise a mixed extract of *Cynanchum wilfordii, Phlomis umbrosa* and *Angelica gigas* or a mixture of *Cynanchum wilfordii* extract, *Phlomis umbrosa* extract and *Angelica gigas* extract.

In the present invention, the *Cynanchum wilfordii* extract may be an extract prepared by using at least one selected from the group of the root, stem and leaf thereof. For example, the extract may be prepared by using the root, but is not limited thereto.

In the present invention, the *Phlomis umbrosa* extract may be an extract prepared by using at least one selected from the group of the root, stem and leaf thereof. For example, the extract may be prepared by using the root, but is not limited thereto.

In the present invention, the *Angelica gigas* extract may be an extract prepared by using at least one selected from the group of the root, stem and leaf thereof. For example, the extract may be prepared by using the root, but is not limited thereto.

The extract of the present invention may be a crude extract obtained by extraction with at least one solvent selected from the group consisting of water and a linear or branched alcohol having 1 to 4 carbon atoms. For example, the extract may be a crude extract obtained by extraction with water as a solvent.

When using a mixture of water and alcohol in one solvent for preparing a crude extract of the present invention, an aqueous solution of linear or branched alcohol having 1 to 4 carbon atoms of 10% or more and less than 100% (v/v), 20% or more and less than 100% (v/v), 30% or more and less than 100% (v/v), 40% or more and less than 100% (v/v), 50% or more and less than 100% (v/v), 60% or more and less than 100% (v/v), or 70% or more and less than 100% (v/v) may be used.

In the present invention, the aqueous alcohol solution may be, but is not limited to, at least one selected from the group consisting of an aqueous methanol solution, an aqueous ethanol solution, an aqueous propanol solution and an aqueous butanol solution.

The extract according to the present invention may be a solvent fraction obtained by fractionating the crude extract with an additional solvent.

In the present invention, the solvent fraction may be a solvent fraction obtained by using at least one solvent selected from the group consisting of ethyl ether, ethyl acetate and butanol. For example, the solvent fraction may be a solvent fraction obtained by using butanol as a solvent.

The content of the extract as an active ingredient in the composition according to the present invention may be appropriately adjusted according to the form and purpose of use, patient conditions, type and severity of symptoms, etc., and may be, but is not limited to, 0.001 to 99.9 wt. % or 0.1 to 99.9 wt. %, preferably 0.1 to 50 wt. % or 0.1 to 40 wt. %.

The composition according to the present invention may be administered to mammals including humans via various routes. The composition may be administered by any method typically used. For example, the composition may be administered via an oral, skin, vein, muscle or subcutaneous route, preferably via an oral route.

The composition of the present invention may be formulated into an oral dosage form such as powders, granules, tablets, capsules, ointments, suspensions, emulsions, syrups, aerosols, etc., or into a parenteral dosage form such as transdermal agents, suppositories and sterile injectable solutions, according to typically used methods.

The composition of the present invention may further comprise an adjuvant such as a pharmaceutically suitable and physiologically acceptable carrier, excipient and diluent, in addition to the mixed extract.

Carriers, excipients and diluents that may be comprised in the composition of the present invention may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate and mineral oil.

When formulating the composition, diluents or excipients such as fillers, extenders, binders, wetting agents, disintegrating agents, surfactants, etc., commonly used, may be used. Solid formulations for oral administration may include tablets, pills, powders, granules, capsules, etc., and may be prepared by adding to the extract at least one excipient, for example, starch, calcium carbonate, sucrose or lactose, gelatin, etc. In addition to simple excipients, lubricants such as magnesium stearate talc may also be used.

Formulations for oral administration are suspensions, oral liquids, emulsions syrups, ointments, etc., and may comprise various excipients, for example, wetting agents, sweeteners, fragrances, preservatives, etc., in addition to commonly used simple diluents, water and liquid paraffin.

Formulations for parenteral administration include sterile aqueous solutions, non-aqueous solvents, suspensions, emulsions, lyophilized preparations, suppositories, transdermal agents, etc. For the non-aqueous solvents and suspensions, propylene glycol, polyethylene glycol, vegetable oils such as olive oil, injectable esters such as ethyl oleate, etc., may be used.

Formulations for suppositories, witepsol, macrogol, tween 61, cacao butter, laurin butter, glycerogelatin, etc., may be used.

In an embodiment in which the composition of the present invention is applied to humans, the composition of the present invention may be administered alone, but may be generally administered in combination with a pharmaceutical carrier selected in consideration of administration methods and standard pharmaceutical practice.

For example, the composition of the present invention may be administered orally, intraorally or sublingually in the form of tablets containing starch or lactose, in the form of capsules containing the composition alone or excipients or in the form of elixirs or suspensions containing chemicals having flavor or color. Such liquid formulations may be formulated in combination with a pharmaceutically acceptable additive such as suspensions (e.g., glyceride mixture such as a mixture of methylcellulose, semi-synthetic glycerides such as witepsol or apricot kernel oil and PEG-6 esters, or a mixture of PEG-8 and caprylic/capric glycerides).

The dose of the composition of the present invention may vary depending on the patient's age, weight, sex, dosage form, medical condition and severity of disease, and may be administered once a day or several times a day in divided doses at regular intervals according to the judgement of a doctor or pharmacist. For example, the daily dose may be 0.1 to 500 mg/kg, preferably 0.5 to 300 mg/kg, based on the content of the active ingredient. The above dose is an example of the average case and may be higher or lower depending on individual differences.

When the daily dose of the composition of the present invention is less than the above amount, a significant effect cannot be obtained, and when it is more than the above amount, it is uneconomical and out of the range of common dose, which may cause undesirable side effects. Thus, it is preferable to set the daily dose within the above range.

In the present invention, the composition induces adenosine A1 agonist activity to exhibit the effect of preventing or treating anxiety disorders or tranquilizing.

GPCRs, target proteins of different ligands, are the group of receptors responsive to various signal transmitters such as neurotransmitters, hormones, etc., and play an important role in regulation of physiological functions such as reproduction, metabolism, immunity, psychological processes, etc., in human. Thus, the GPCR profiling was performed to screen the GPCRs which are affected by the composition of the present invention, assess the dose response on adenosine A1, beta-3 adrenergic receptor, adenosine A2A and GABAA ion channel, which are known as the mechanism of action associated with tranquilizing, and perform the tracking evaluation of the mechanism of action. As a result, it was confirmed that the extract of the present invention induces the activity of concentration dependent adenosine A1 agonist to improve anxiety disorders, and that other adenosine A2A, beta-3 adrenergic receptor and GABAA ion channel are not associated therewith.

Another embodiment of the present invention relates to a food composition for preventing or improving an anxiety disorder, comprising at least one extract selected from the group consisting of *Cynanchum wilfordii, Phlomis umbrosa* and *Angelica gigas.*

In the present invention, the anxiety disorder may be, but is not limited to, separation or isolation anxiety disorder, paranoia, panphobia, generalized obsessive-compulsive disorder, anxiety neurosis or panic disorder.

Another embodiment of the present invention relates to a food composition for tranquilizing, comprising at least one extract selected from the group consisting of *Cynanchum wilfordii, Phlomis umbrosa* and *Angelica gigas.*

In the present invention, the food composition for tranquilizing may be for preventing or improving bipolar disorder, neurasthenia or schizophrenia, but is not limited thereto.

The extract is the same as described above, and thus the description thereon is omitted.

The health functional food may be various foods, beverages, food additives, etc.

The content of the herbal extract as an active ingredient contained in the health functional food is not particularly limited according to the form of food, desired use, etc., and for example, may be added in an amount of 0.01 to 15 wt. % of the total food weight, and a health beverage composition may be added in the proportion of 0.02 to 10 g, preferably 0.3 to 1 g based on 100 ml.

The health beverage composition of the present invention has no particular limitation on the liquid ingredient except that the composition contains the extract as the essential ingredient in the designated proportion, and may contain various flavoring agents or natural carbohydrates as additional ingredients, like ordinary drinks.

The natural carbohydrates include typical saccharides, such as monosaccharides, for example, glucose, fructose, etc., disaccharides, for example, maltose, sucrose, etc., and polysaccharides, for example, dextrin, cyclodextrin, etc., and sugar alcohols such as xylitol, sorbitol, erythritol, etc.

As flavoring agents other than those described above, natural flavoring agents (thaumatin, stevia extract (e.g., rebaudioside A, glycyrrhizin, etc.) and synthetic flavoring agents (saccharin, aspartame, etc.) may be advantageously used. The proportion of the natural carbohydrates is generally about 1 to 20 g, preferably about 5 to 12 g per 100 ml of the composition of the present invention.

In addition to the above, the composition of the present invention may contain various nutrients, vitamins, minerals (electrolytes), flavoring agents such as synthetic flavoring agents and natural flavoring agents, coloring and enhancing agents (cheese, chocolate, etc.), pectic acid and salts thereof, alginic acid and salts thereof, organic acids, protective colloidal thickeners, pH regulators, stabilizers, preservatives, glycerin, alcohols, carbonating agents used in carbonated drinks, etc. In addition, the composition of the present invention may contain flesh for preparing natural fruit juices, fruit juicy beverages and vegetable beverages. These ingredients may be used independently or in combination. The proportion of the additives is not so critical but is generally selected in the range of 0 to about 20 parts by weight per 100 parts by weight of the composition of the present invention.

In particular, when the extract of the present invention is administered to the human body, it is considered that there are no concerns about side effects compared to other synthetic pharmaceuticals in view of the general characteristics of natural extracts. Actually, as a result of toxicity tests on the standardized herbal composition, it was determined that the extract has no impact on the human body.

A process for preparing the extract according to the present invention is explained in more detail as follows.

After cutting at least one selected from the group consisting of *Cynanchum wilfordii, Phlomis umbrosa* and *Angelica gigas,* washing the same with water to remove impurities, and drying the same, reflux extraction is conducted with an extraction solvent of about 5 to 20 times by volume, preferably 7 to 15 times by volume relative to the weight of the at least one selected from the group consisting of *Cynanchum wilfordii, Phlomis umbrosa* and *Angelica gigas.* After extraction, the filtrate is collected by filtration. The extraction temperature is not particularly limited, but is 40 to 110°C, preferably 55 to 105°C.

The extraction process may be repeated once or several times. In a preferred embodiment of the present invention, a method of re-extracting after primary extraction may be adopted. This is because when the herbal extract is mass-produced, loss occurs because of the high water content of the herb itself although effective filtration is performed, and thus lower extraction efficiency is obtained from the primary extraction alone. In addition, as a result of verifying the extraction efficiency for each step, it was found that about 80 to 90% of the total extraction amount is extracted by the secondary extraction.

In an embodiment of the present application, when the extraction process is repeated twice, the obtained residue is extracted under reflux again with the extraction solvent of about 5 to 15 times by volume, preferably 8 to 12 times by volume. After extraction, filtration is performed, and the filtrate is mixed with the filtrate previously obtained. The mixture is concentrated under reduced pressure, to prepare an extract.

As such, the extraction efficiency may be increased by twice extraction and mixing the filtrates obtained after each extraction, however, the extract of the present invention is not limited to the number of times of extraction.

When the amount of the solvent used for preparing the extract of the present invention is too small, it is difficult to stir, and the solubility of the extract is lowered, which results in a decrease in extraction efficiency. When it is too large, the amount of the solvent to be used in the next purification step is large, which is not economical and causes handling problems. Thus, the amount of the solvent to be used is preferably within the above range.

In order to adjust the content of residual lower alcohols such that the extract is suitable for the use as a pharmaceutical or food material, the filtered extract thus obtained is azeotropically concentrated once to five times, preferably twice to three times with water of about 10 to 30 times, preferably 15 to 25 times, more preferably about 20 times by weight of the total amount of the concentrate, and water in the same amount is added again to homogeneously suspend the concentrate, followed by lyophilization and/or spray drying, to prepare an extract in the form of powders.

The extraction of the present invention may be carried out by any method typically used. For example, extraction methods may include maceration extraction, hot water extraction, ultrasonic extraction or reflux extraction. For example, the extraction method may be, but is not limited to, hot water extraction.

**Effect of invention**

The present invention relates to a composition for improving, preventing or treating an anxiety disorder, and a tranquilizing composition.

**Brief description of drawings**

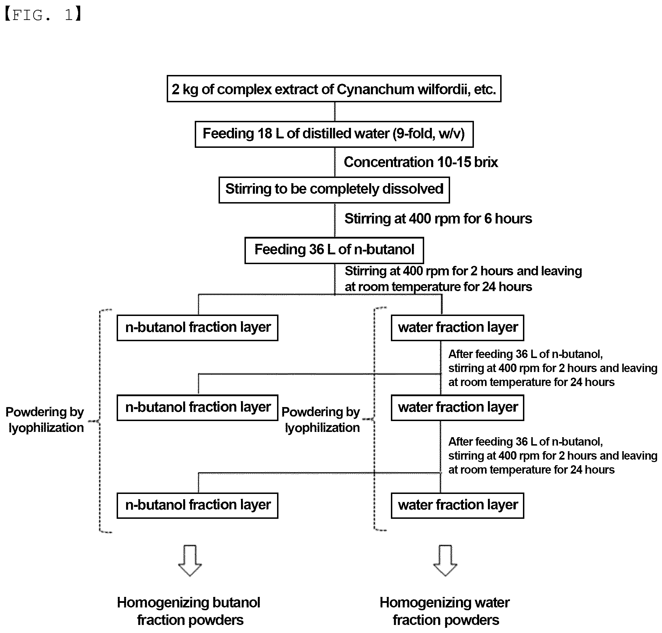

Fig. 1 is a schematic diagram of preparation of the fraction according to an embodiment of the present invention;
Fig. 2 is a graph showing the measurement result of tranquilization induction time of the crude extract according to an embodiment of the present invention;
Fig. 3 is a graph showing the measurement result of tranquilization induction time of the butanol fraction according to an embodiment of the present invention;
Fig. 4 is a graph showing the measurement result of tranquilization duration of the crude extract according to an embodiment of the present invention;

Fig. 5 is a graph showing the measurement result of tranquilization duration of the butanol fraction according to an embodiment of the present invention; and

Fig. 6 is a graph showing the evaluation result of the crude extract and butanol fraction against anxiety disorders according to an embodiment of the present invention.

## Best mode for carrying out the invention

A pharmaceutical composition for preventing or treating an anxiety disorder, comprising at least one extract selected from the group consisting of *Cynanchum wilfordii, Phlomis umbrosa* and *Angelica gigas.*

## Modes for carrying out the invention

Hereinafter, the present invention will be described in detail with reference to the following embodiments. However, the embodiments are only for exemplifying the present invention, and the present invention is not limited to the embodiments.

## Example 1. Preparation of Crude Extract

After mixing natural medicinal herbs, the roots of *Cynanchum wilfordii,* the roots of *Phlomis umbrosa* and the roots of *Angelica gigas,* in a weight ratio of 1:1:1.08, the mixture was heated with 10 fold water at a temperature of 95 to 105°C for 8 hours and extracted. Then, the filtrate obtained by filtration was lyophilized at -80°C, to obtain a crude extract in powder form.

## Example 2. Preparation of Butanol Fraction of Crude Extract

2 kg of the crude extract in powder form of Example 1 were dissolved with water to have 10 to 15 brix, fed into a large-scale stirrer, and stirred at 400 rpm for 6 hours, to obtain an aqueous solution of the crude extract. Then, n-butanol in an amount corresponding to 2 fold (v/v) of the aqueous solution of the crude extract was fed thereinto, stirred at about 400 rpm for 2 hours, and left standing at room temperature for 24 hours or more, to perform a liquid-liquid fraction. After the liquid-liquid fraction, the top clear n-butanol layer was carefully collected, and the liquid-liquid fraction and collection process was repeated twice for the bottom water layer. Thereafter, only the n-butanol layer was collected and the solvent was completely removed using a vacuum concentrator to have 30 to 33 brix, and then the concentrate was dissolved sufficiently with distilled water to have 13 to 15 brix, followed by lyophilization. The powders obtained after completion of lyophilization were ground with a blender and sifted through a 500 mesh sieve for homogenization, and then left in a desiccator under reduced pressure (25°C, -0.76 mbar) for 8 hours or more, to be double packaged and stored.

## Experimental Example 1. Measurement of Tranquilization Induction Time

## 1-1. Crude extract

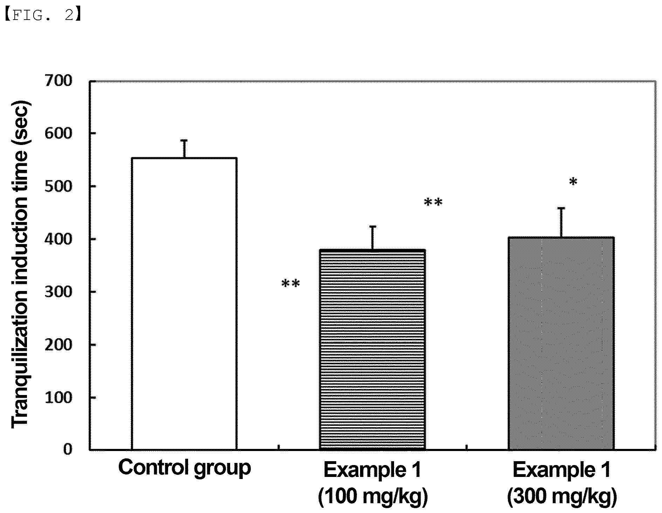

The efficacy was proved through the pentobarbital-induced tranquilization model in mice. Specifically, after 1 week acclimation, the composition of Example 1 was repeatedly administered once daily for 4 weeks in total. The composition of the example was taken in an amount of 100 or 300 mg/kg. After 30 minutes from the last intake, the tranquilization inducer pentobarbital 40 mg/kg was administered, and the tranquilization induction time was measured. The result was shown in Fig. 2 and Table 1.

[Table 1]

| Group | Tranquilization induction time (sec.) |
|---|---|
| Control group | $553.25 \pm 33.67$ |
| Example 1 (100mg/kg) | $379.38 \pm 44.30$** |
| Example 1 (300mg/kg) | $403.00 \pm 54.96$* |
| Statistically significant control group *: $p < 0.05$, **: $p < 0.01$ | |

As can be confirmed from Fig. 2 and Table 1, it was confirmed that when compared with the control group, the tranquilization induction time was statistically significantly reduced in both of the two dose groups of the composition of Example 1 (100 mg/kg intake group: p<0.01, 300 mg/kg intake group: p<0.05).

**1-2. Butanol fraction**

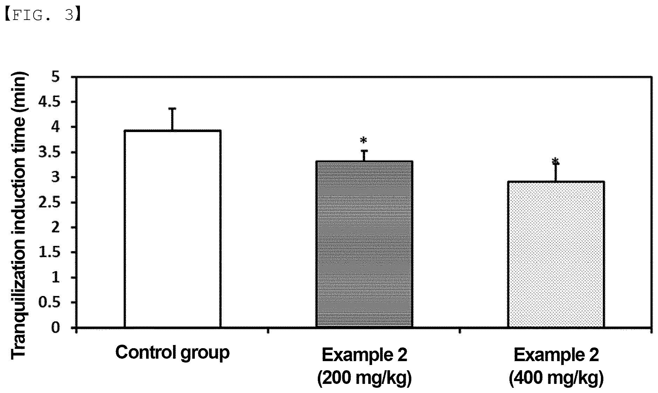

The efficacy was proved through the pentobarbital-induced tranquilization model in mice. Specifically, after 1 week acclimation, the composition of Example 2 was repeatedly administered once daily for 2 weeks in total. The composition of Example 2 was taken in an amount of 200 or 400 mg/kg. After 45 minutes from the last intake, the tranquilization inducer pentobarbital 45 mg/kg was administered, and the tranquilization induction time was measured. The result was shown in Fig. 3 and Table 2.

[Table 2]

| Group | Tranquilization induction time (min.) |
|---|---|
| Control group | 3.926±0.442018 |
| Example 2 (200mg/kg) | 3.31±0.221359436* |
| Example 2 (400mg/kg) | 2.902±0.364032966* |
| Statistically significant control group *: p<0.05, **: p<0.01 | |

As can be confirmed from Fig. 3 and Table 2, it was confirmed that when compared with the control group, the tranquilization induction time was statistically significantly reduced in both of the dose groups 200 mg/kg and 400 mg/kg of Example 2 (p<0.05).

**Experimental Example 2. Measurement of Tranquilization Duration**

**2-1. Crude extract**

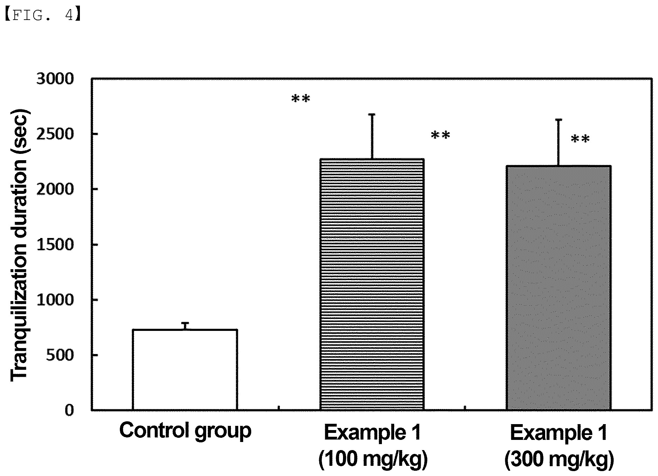

After inducing tranquilization in Experimental Example 1-1, the time in which nerves are activated was measured for each group, and the duration of tranquilization was measured. The result was shown in Fig. 4 and Table 3.

[Table 3]

| Group | Tranquilization duration (sec.) |
|---|---|
| Control group | 727.63±65.73 |
| Example 1 (100mg/kg) | 2274.50±404.42** |
| Example 1 (300mg/kg) | 2207.13±418.52** |
| Statistically significant control group *: p<0.05, **: p<0.01 | |

As can be confirmed from Fig. 4 and Table 3, it was confirmed that the duration of tranquilization was statistically significantly increased in both of the two dose groups of the composition of the example (p<0.01).

**2-2. Butanol fraction**

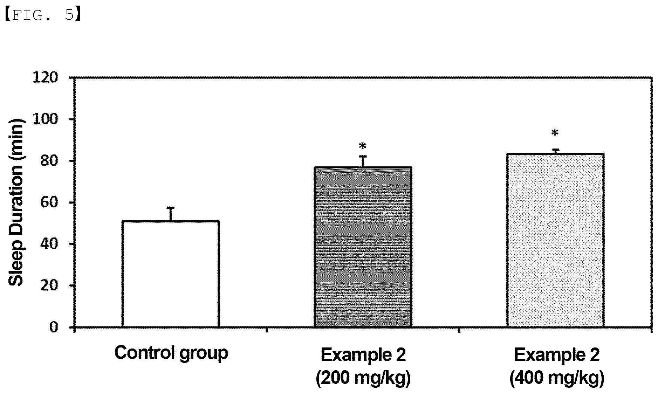

After inducing tranquilization in Experimental Example 1-2, the time in which nerves are activated was measured for each group, and the duration of tranquilization was measured. The result was shown in Fig. 5 and Table 4.

[Table 4]

| Group | Tranquilization duration (min.) |
|---|---|
| Control group | 50.73±6.845363 |
| Example 2 (200mg/kg) | 76.862±5.359339512* |
| Example 2 (400mg/kg) | 83.456±1.945412553* |
| Statistically significant control group *: p<0.05, **: p<0.01 | |

As can be confirmed from Fig. 5 and Table 4, it was confirmed that the duration of tranquilization was statistically significantly increased in both of the two dose groups of Example 2 (p<0.05).

**Experimental Example 3. Evaluation of Tranquilization Induction at Titer or less**

The tranquilization inducer pentobarbital 30 mg/kg that is smaller than or equal to the titer was administered, to evaluate the rate of tranquilization induction in the tranquilization borderline state. The result was shown in Table 5.

[Table 5]

| Group | Dose (mg/kg) | Tranquilization rate (%) | Tranquilization time (min.) |
|---|---|---|---|
| Control group | - | 20 | 6.52 |
| Example | 10 | 30 | 10.37 |
| | 100 | 60 | 13.02 |
| | 200 | 50 | 11.27 |
| | 400 | 50 | 10.59 |

Tranquilization rate (%) = number of subjects with activated nerves / total number of subjects X 100

As can be confirmed from Table 5, the tranquilization induction rate was about 20% in the non-treated group, whereas the tranquilization induction rate was about 60 to 50% in the groups administered with the composition of the example in an amount of 100 mg/kg or more, and particularly better effect was shown in increasing the duration of tranquilization. Thus, it was confirmed that the composition of the example has the effect of inducing tranquilization.

Consequently, regarding the improvement of anxiety disorders, it was confirmed that the composition of the example statistically significantly improves both of the tranquilization induction time and the tranquilization duration, which are the associated markers, in the pentobarbital-induced tranquilization animal model, and has the excellent effect of improving anxiety disorders, when compared with the control group.

**Experimental Example 4. Evaluation of Mechanism of Action associated with Tranquilizing (GPCR)**

In order to define the mechanism of action on improvement of anxiety disorders, the pattern of GPCR activity associated with tranquilizing was analyzed, and a profiling assay was performed to find the mechanism of action point showing the effect.

GPCRs, target proteins of different ligands, are the group of receptors responsive to various signal transmitters such as neurotransmitters, hormones, etc., and play an important role in regulation of physiological functions such as reproduction, metabolism, immunity, psychological processes, etc., in human. Thus, the GPCR profiling was performed to screen the GPCRs which are affected by the composition of the example, assess the dose response on adenosine A1, beta-3 adrenergic receptor, adenosine A2A and GABAA ion channel, which are known as the mechanism of action associated with tranquilizing, and perform the tracking evaluation of the mechanism of action.

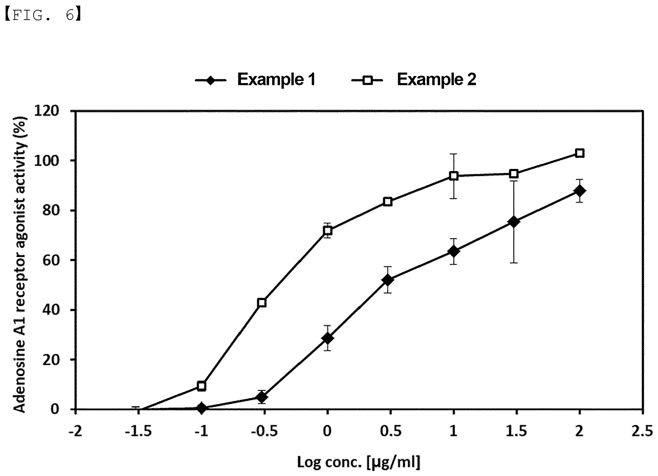

Specifically, in order to see the dose-response mechanism of the extracts, the test was carried out by serial dilution with 8 concentrations including the highest concentration 100 ug/ml. When the effective maximum value of a reference agonist for each known receptor is calculated as 100 percent, a GPCR having 50% or higher activity obtained by converting the activity compared therewith into percent (%), may be determined to have activity as an agonist. For the adenosine A1 receptor, a calcium fluorimetry assay was performed in human recombinant BA/F3 cells in which the stable expression of A1 receptor is induced, and the activity thereof was evaluated by comparison with when the effective maximum value of the adenosine A1 receptor agonist CPA is calculated as 100 percent. The result was shown in Fig. 6 and Table 6.

[Table 6]

| Concentration (ug/ml) | Example 1 (%) | Example 2 (%) |
|---|---|---|
| 0.03 | -0.1±1.2 | -0.8±1.6 |

(continued)

| Concentration (ug/ml) | Example 1 (%) | Example 2 (%) |
|---|---|---|
| 0.1 | $0.5\pm1.1$ | $9.5\pm2.1$ |
| 0.3 | $5\pm2.5$ | $42.8\pm0.7$ |
| 1 | $28.7\pm5.2$ | $72\pm2.9$ |
| 3 | $52\pm5.3$ | $83.4\pm0.6$ |
| 10 | $63.5\pm5.3$ | $93.7\pm9.1$ |
| 30 | $75.4\pm16.5$ | $94.7\pm1.6$ |
| 100 | $87.9\pm4.6$ | $102.9\pm1.08$ |

As can be seen from Fig. 6 and Table 6, as the concentrations of the extracts of Example 1 and the butanol fractions thereof of Example 2 increase to 0.03, 0.1, 0.3, 1, 3, 10, 30, and 100 ug/ml, the extracts and the butanol fractions thereof showed the concentration dependent agonist activity. The extracts of Example 1 at the concentration of 3 ug/ml or more and the extracts of Example 2 at the concentration of 1 ug/ml or more showed high activity of 50% or higher. It was confirmed that among the mechanisms of action associated with tranquilizing, the composition of the example induces the activity of concentration dependent adenosine A1 agonist to improve anxiety disorders, and that other adenosine A2A, beta-3 adrenergic receptor and GABAA ion channel are not associated therewith. Accordingly, the mechanism of action on improvement of anxiety disorders was obtained. In addition, the strong agonist activity for adenosine A1 receptor was confirmed, and the 50% effective concentration (EC50) was exhibited in the composition at 2.15 ug/ml of Example 1 and the composition at 0.33 ug/ml of Example 2.

## Industrial applicability

The present invention relates to a composition for improving, preventing or treating an anxiety disorder, and a tranquilizing composition.

## Claims

1. A pharmaceutical composition for preventing or treating an anxiety disorder, comprising at least one extract selected from the group consisting of *Cynanchum wilfordii, Phlomis umbrosa* and *Angelica gigas.*

2. The pharmaceutical composition of claim 1, wherein the anxiety disorder is separation or isolation anxiety disorder, paranoia, panphobia, generalized obsessive-compulsive disorder, anxiety neurosis or panic disorder.

3. The pharmaceutical composition of claim 1, wherein the extract is a crude extract obtained by extraction with at least one solvent selected from the group consisting of water and a linear or branched alcohol having 1 to 4 carbon atoms.

4. The pharmaceutical composition of claim 1, wherein the extract is a solvent fraction obtained by extracting a crude extract obtained by extraction with at least one solvent selected from the group consisting of water and a linear or branched alcohol having 1 to 4 carbon atoms with at least one solvent selected from the group consisting of ethyl ether, ethyl acetate and butanol.

5. A food composition for preventing or improving an anxiety disorder, comprising at least one extract selected from the group consisting of *Cynanchum wilfordii, Phlomis umbrosa* and *Angelica gigas.*

6. The food composition of claim 5, wherein the anxiety disorder is separation or isolation anxiety disorder, paranoia, panphobia, generalized obsessive-compulsive disorder, anxiety neurosis or panic disorder.

7. A pharmaceutical composition for tranquilizing, comprising at least one extract selected from the group consisting of *Cynanchum wilfordii, Phlomis umbrosa* and *Angelica gigas.*

**8.** The pharmaceutical composition of claim 7, wherein the pharmaceutical composition for tranquilizing is for treating or preventing bipolar disorder, neurasthenia or schizophrenia.

**9.** A food composition for tranquilizing, comprising at least one extract selected from the group consisting of *Cynanchum wilfordii, Phlomis umbrosa* and *Angelica gigas.*

**10.** The food composition of claim 9, wherein the food composition is for treating or preventing bipolar disorder, neurasthenia or schizophrenia.

【FIG. 1】

```
          ┌──────────────────────────────────────────────────┐
          │ 2 kg of complex extract of Cynanchum wilfordii, etc. │
          └──────────────────────────────────────────────────┘
                              │
          ┌──────────────────────────────────────────┐
          │ Feeding 18 L of distilled water (9-fold, w/v) │
          └──────────────────────────────────────────┘
                              │  Concentration 10-15 brix
          ┌──────────────────────────────────┐
          │ Stirring to be completely dissolved │
          └──────────────────────────────────┘
                              │  Stirring at 400 rpm for 6 hours
          ┌──────────────────────────┐
          │ Feeding 36 L of n-butanol │
          └──────────────────────────┘
                              │  Stirring at 400 rpm for 2 hours and leaving
                              │  at room temperature for 24 hours
```

| n-butanol fraction layer | water fraction layer |

After feeding 36 L of n-butanol,
stirring at 400 rpm for 2 hours and leaving
at room temperature for 24 hours

Powdering by lyophilization

| n-butanol fraction layer | water fraction layer |

Powdering by lyophilization

After feeding 36 L of n-butanol,
stirring at 400 rpm for 2 hours and leaving
at room temperature for 24 hours

| n-butanol fraction layer | water fraction layer |

⇩ Homogenizing butanol fraction powders

⇩ Homogenizing water fraction powders

【FIG. 2】

【FIG. 3】

【FIG. 4】

【FIG. 5】

【FIG. 6】

## INTERNATIONAL SEARCH REPORT

| International application No. |
|---|
| **PCT/KR2021/003081** |

| **A.** | **CLASSIFICATION OF SUBJECT MATTER** |
|---|---|
| | **A61K 36/27**(2006.01)i; **A61K 36/53**(2006.01)i; **A61K 36/232**(2006.01)i; **A61P 25/22**(2006.01)i; **A23L 33/105**(2016.01)i |

According to International Patent Classification (IPC) or to both national classification and IPC

| **B.** | **FIELDS SEARCHED** |
|---|---|

Minimum documentation searched (classification system followed by classification symbols)

A61K 36/27(2006.01); A23L 1/30(2006.01); A61K 36/481(2006.01); A61K 8/97(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 불안장애(Anxiety disorder), 백수오(Cynanchum wilfordii), 속단(Phlomis umbrosa), 당귀(Angelica gigas), 추출물(extract)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| X | CHANG, A. et al. The Effect of Herbal Extract (EstroG-100) on Pre-, Peri- and Post-Menopausal Women : A Randomized Double-blind, Placebo-controlled Study. Phytotherapy Research. 2012, vol. 26, pp. 510-516. <br> See abstract; table 2; and pages 510, 511 and 516. | 1-10 |
| A | KR 10-2019-0022049 A (KOREA INSTITUTE OF SCIENCE AND TECHNOLOGY) 06 March 2019 (2019-03-06) <br> See claims 1, 8 and 9; and paragraph [0029]. | 1-10 |
| A | 한송희 등. 백수오와 한속단 추출물의 비에스트로젠 효과에 관한 연구. Korean J. Food SCI. Technol. 2015, vol. 47, no. 5, pp. 667-672 (HAN, Song-Hee et al. Mixture of Extracts of Cynanchum wilfordii and Phlomis umbrosa Turcz. Does Not Have an Estrogenic Effect in Ovariectomized Rats.). <br> See page 667, right column, lines 3-8. | 1-10 |
| A | YOON, D. W. et al. Effects of Cynanchum Wilfordii Hemsley Extract on the Sleep-Wake Architectures in Rats. Sleep Medicine Research. 2011, vol. 2, no. 1, pp. 16-20. <br> See page 16. | 1-10 |

☑ Further documents are listed in the continuation of Box C.  ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **20 July 2021** | **20 July 2021** |

| Name and mailing address of the ISA/KR | Authorized officer |
|---|---|
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

# EP 4 122 479 A1

**INTERNATIONAL SEARCH REPORT**

International application No.

**PCT/KR2021/003081**

## C.    DOCUMENTS CONSIDERED TO BE RELEVANT

| Category* | Citation of document, with indication, where appropriate, of the relevant passages | Relevant to claim No. |
|---|---|---|
| A | KR 10-2017-0109703 A (UNIVERSITY-INDUSTRY COOPERATION GROUP OF KYUNG HEE UNIVERSITY) 10 October 2017 (2017-10-10)<br>See claims 1 and 2. | 1-10 |

Form PCT/ISA/210 (second sheet) (July 2019)

19

**INTERNATIONAL SEARCH REPORT**
Information on patent family members

International application No.

**PCT/KR2021/003081**

| Patent document cited in search report | Publication date (day/month/year) | Patent family member(s) | Publication date (day/month/year) |
|---|---|---|---|
| KR   10-2019-0022049   A | 06 March 2019 | None | |
| KR   10-2017-0109703   A | 10 October 2017 | None | |

Form PCT/ISA/210 (patent family annex) (July 2019)